# EUROPEAN PATENT APPLICATION

(11) **EP 2 769 693 A1**
(43) Date of publication of application: **27.08.2014**
(21) Application number: 13005441.4
(22) Date of filing: 19.11.2013
(51) Int. Cl.: A61B 17/84, A61F 2/08, A61B 17/06, A61B 17/064, A61B 17/04

(54) **Lock clamp used in orthopedic surgeries**

(30) Priority: 22.02.2013 TR 201302135
(71) Applicant: Aksamoglu, Mustafa Hilmi, Seyhan - Adana 01130 (TR)
(72) Inventor: Aksamoglu, Mustafa Hilmi, Seyhan - Adana 01130 (TR)
(74) Representative: Gaißmayer, Jörg

(57) **Abstract**

The invention is the lock clamp used in orthopedic surgeries and tip (5) of which is different by the tissue for which it shall be used. The clamp comprises of clamp cable (2) surface of which is covered by reverse threads (3), reverse threads (3) covering the surface of clamp cable (2), tip (5) bottom of which varies by the use area, head (4) and lock clip (1).

## Description

The invention is related to a new and practical lock apparatus to be used for repair fracture, rupture or laceration of bone or soft tissues to be used in orthopedic surgeries.

The clamp can be used for follows:
- Repair of tendon laceration
- Fixing the muscle or tendon parting from adherence point
- Attaching to bone fragments to each other for avulsion fractures
- Necessary fixing by covering the bond like a ring
- Hanging the tendon graft inserted in a tunnel opened in the bone to bone cortex for anterior cruciate ligament or similar ligament injuries
- Fixing to soft tissues such as meniscus to each other

Standard techniques applied related to every intended use are as follows:
- Tendon lacerations are stitched with surgical sutures by means of various suture techniques and kept in splint for a length of time following the surgery.
- Tendon or muscles breaking from the bone are fastened by stitching with sutures on apparatus in the form of screw or anchor. Movement is restricted for relevant joints for a given period.
- To fix the bone fragments in the event of avulsion fractures, screws or wires are used.
- In the cases that the bone body should be wound, cerclage wire or cable is used.
- Tendon graft is used for ligament reconstructions. This tendon graft is passed through a ring. The end of ring is taken above the bone cortex via a tunnel opened in the bone and fixed by hanging to this section.
- For fixing the tissues such as meniscus, ready sliding knotted sutures for which there is a polyethylene bead in the end are used.

In the present techniques:
- Surgical procedure implemented for repairing tendons last long. In the event of multiple tendon lacerations such as wrist lacerations the time of surgery may be 4-5 hours. Moreover, since the power of sutures is not enough for active use, the limb needs to be immobile or partially immobile for the time as the expected recovery time of the tendon (generally 3-6 weeks). In this case, flexor tendons especially in the hand suffer from adhesion in their own cuffs. The cases that the movement cannot be achieved completely following the recovery are seen in high rates.
- The operation of fixing the muscle or tendon parting from the bone again is a very difficult surgical operation. This difficulty has been overcome significantly by means of screw or anchor type fixing tools. However, these apparatus only enable the fixing to the bone. Sutures are used for fixing muscle or tendon. These should be passed through suitable sections and knotted. The fixing strength of the knots is limited. Knots sliding allow the muscle to place on the bone surfaces of the muscle with low pressure and on small space. The level of pressure implemented on the bone surface with the suture is proportional to the success of the fixing. Many techniques were described to increase the pressure and contact area. Unsuccessful fixings are high in number due to insufficient pressure.
- Wire can be used to fix the bone fragments for avulsion fractures. It is an easy operation. However, it fixes the bone fragments in the place. It does not lead to compression by pulling them to each other. For this reason, its success is very limited. In the cases that screws are used, the technique is relatively difficult. It may lead to limited compression. If the fragment is not very big, it can be broken by screw. The fixing on the main fragment other than the fragment avulsed is sometimes insufficient.
- If the diaphysis is wound by cerclage, periost membrane feeding the bone may be crushed and bone tissue may be damaged. For this reason, cables with more effects are used. For the fixing with cables, sliding and insufficiency of fixing is very frequently seen.
- The tunnel in tendon thickness required to place the hanging ring on the cortex used for ligament reconstructions should be opened minimum 10 mm more than the required one. After the tendon is fit into place, 10 mm will be empty in the tunnel. This gap leads to oscillation movement in the tendon hung. And it may deteriorate the process of joining with the bone.
- A long learning process is necessary for implementing meniscus sutures and these implants are generally expensive.

The implementation of lock clamp developed to solve these problems is easier than all available methods. It may be learnt and implemented very fast. It is sounder than surgical sutures. It is more economic than the alternative products.

The advantages of the clamp being the subject matter of the invention by the areas of use are as follows:
- The time of repair of a flexor tendon which can be repaired in 20 minutes as the fastest for tendon lacerations is maximum 5 minutes with this product. Since it is a very sound fixing, it needs no splint. It allows active action within one two days and no adhesion occurs. The function can be recovered completely.
- Following placing screw or anchor in the bone, the cable of the clamp should pass through the muscle 1 time. Pressing the clip on the bone by pushing lasts for a few seconds. Hundreds times more pressure can be exerted on minimum 100 times large area by any suture knot. The muscle should not be crushed.
- Even if the bone fragments are small or big, they can caught with the clamp easily and fixing is achieved with very high compression and very fast. Involvement to the main fragment is of very high endurance.
- Diaphysis can be wound easily. No crushing is in question by means of ducts on the periost membrane following the fixing. The risk of insufficient fixing and sliding due to lug to be added will be eliminated.
- Tendon fits in the tunnel opened well by means of the clamp. Since there is no gap, oscillation motion will not be seen. Moreover, joining of bone tendon will be sounder.
- Repair of meniscus rupture will be easier, faster and sounder with this method. This product can be applied with the internal technique or outside in technique for meniscus suturing.

Clamp is produced in six different tip constructions by the use area. These are as follows:
- Knob tip: for repair of tendon lacerations
- Screw tip: fixing the tendons or muscles parted from the adhesion point to the bone.
- Hard collector wing tip: attaching the bone fragments for avulsion fractures to each other
- Ring tip: necessary fixing by winding the bone like a circle.
- Wide and oval ring tip: for hanging the tendon graft placed in a tunnel opened in the bone for anterior cruciate ligament or similar ligament injuries to the bone cortex
- Soft collective tip: for fixing soft tissues like meniscus to each other.

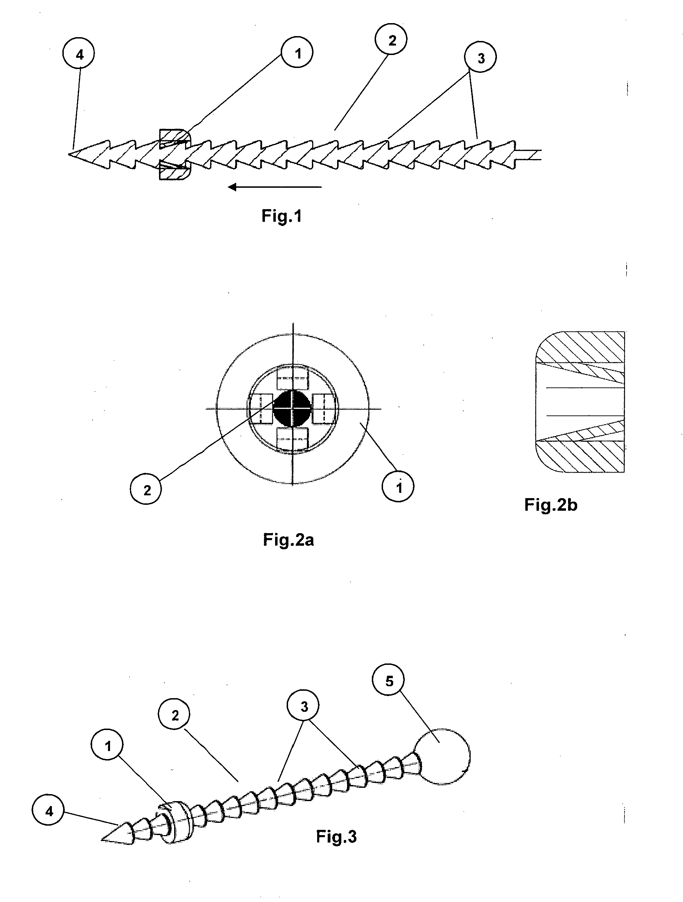

The clamp tip of which differentiates by the different use area is shown in the figures attached and the explanations of the figures are as follows:
Figure -1: general perspective view of the clamp
Figure 2a- sectional view of the clamp
Figure 2b- sectional view of the clamp at the level of lock
Figure 3- perspective view of the knob tip clamp

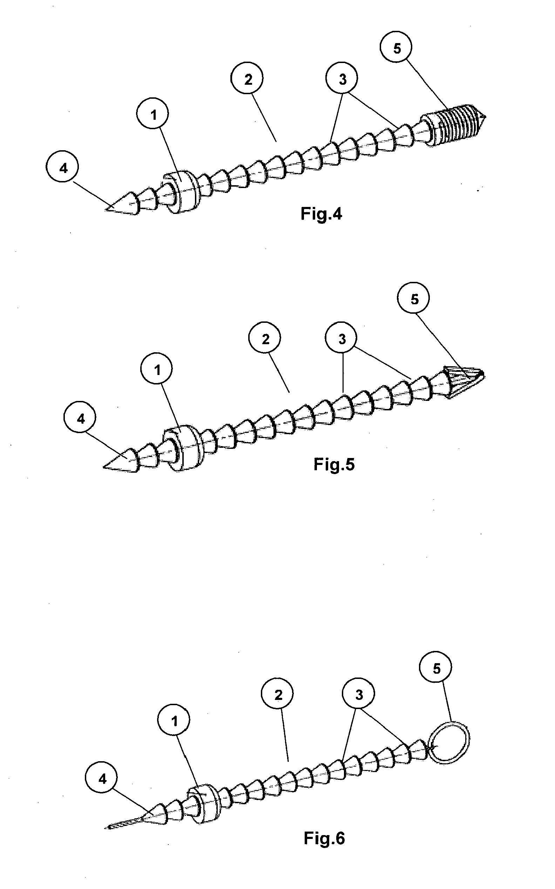

Figure 4- perspective view of the screw tip clamp
Figure 5- perspective view of the hard collector wing tip clamp
Figure 6- perspective view of the ring tip clamp

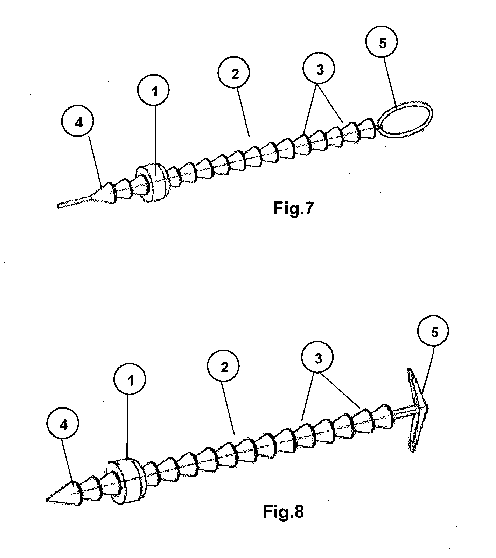

Figure 7- perspective view of the wide and oval ring tip clamp
Figure 8- perspective view of the soft collector tip clamp

Parts in the figures are numbered one by one and equivalents of the numbers are as follows:
1- Lock clip
2- Clamp cable
3- Reverse threads
4- Head
5- Tip

The clamp produced in different size and different tip constructions by the use area is made of plastic type materials not leading to reaction in the body and used for propylene derivative sutures or different materials with the similar functional characteristic. Metal frame may be required in some cases by the use area. In this case, nitinol or metal with similar characteristics is used.

Lock clamp system is used instead of suture and sliding knot for fixing the soft tissue. Clamp cable (2) is thicker and sounder than the suture. Breaking strength is higher than the suture. Clip (1) locking is safer and faster than the suture knot. Since the placement base is wide, it allows sound fixing equivalent to a few knots tied side by side.

Clamp cable (2) is surrounded by reverse threads (3) moving in only one direction and not allowing turning back. The locking is achieved by a clip (1) which stays in the area it is directed by sliding easily towards only one direction and is locked since it is attached to the large surface of the threads on the cable and cannot go back due to the protrusions in its internal structure. The head part (4) can pass through the lock clip (1) easily and the tip (5) can be produced in different forms depending on the tissue for which the apparatus will be used.

Mechanism of the clamp allows movement in only one direction. In practice, clamp cable (2) on which reverse threads (3) are available folds on the tissue by means of flexible construction and tissue is compressed between the tip (5) and lock clip (1) and fixed.

When the lock clamp system is placed on a metal frame, screw is used instead of cable and wire.

Metal frame lock clamp system allows compression not being in question for the wire by the effect of clip (1). It is more resistant to sliding comparing to the cable. Screws can grip certain sizes of fragments. Smaller fragments can be fixed more safely in this system.

The clamp tip (5) of which is different for every use area can be produced in series in the relevant field of the industry.

## Claims

1. It is the lock clamp to be used for repairing fracture, laceration or incision of bone or soft tissues and it comprises of clamp cable (2) which covers the tissue b folding on the tissue owing to its flexible structure and surface of which is covered by reverse threads (3), reverse threads (3) which allow only single-direction movement and covers the surface, tip part (5) produced in six different forms by the use area, head part (4) which allows easy pass of the cable (3) through the lock clip (1) and lock clip (1) which stays in the area it is directed by sliding easily towards only one direction and is locked since it is attached to the large surface of the threads on the cable and cannot go back due to the protrusions in its internal structure.
